# EUROPEAN PATENT APPLICATION

(11) **EP 1 477 586 A2**
(43) Date of publication of application: **17.11.2004**
(21) Application number: 04011275.7
(22) Date of filing: 12.05.2004
(51) Int. Cl.: C25D 3/04, C25D 5/34, A61F 2/32

(54) **A movable joint and method for coating movable joints**

(30) Priority: 12.05.2003 US 435813
(71) Applicant: Noble Medical Coatings, L.L.C., Cleveland, Ohio 44110 (US)
(72) Inventor: Corl III, Harry E., Twinsburg Ohio 44087 (US); Noble, Lawrence E., Scottsdale Arizona 85258 (US)
(74) Representative: Kirschner, Klaus Dieter, Dipl.-Phys.

(57) **Abstract**

In a process of electroplating a metal work piece with thin dense chromium, the steps of submerging the metal work piece in a 35% sulfuric acid solution having about 4 ounces per gallon HF as ammonium biflouride salts, and subsequently submerging the metal work piece in a thin dense chromium plating bath having an initial direct current of about 3 volts, the amperages being at about 1.5-2.5 amps per square inch of cathode area, is provided.

## Description

The present invention relates generally to the field of movable joints.

Movable joints have been utilized in many different technical areas, from medical implants to automobile parts, with each technical area having different, important characteristics. In some applications, the amount of constant load that a joint can maintain over a long duration is important. In other applications, the amount of extreme load that a joint can maintain over a short period of time may be important. In still other applications, the wear resistance of the joint when the parts of the joint are in relatively constant movement is important. Most applications require a mix of these important factors.

One such application is the use of a ball-type joint to replace a natural joint in a human or animal. Ball joints have proven useful in this application because, like the natural joint that the implant is replacing, the joint provides a wide range of motion. However, under these conditions, it is important to have a joint that can be in relatively constant motion and exposed to differing loads without becoming worn, thereby, requiring the joint to be replaced. Since the replacement of the joint is accomplished through invasive surgery, the longer the joint can be utilized without repair or replacement the less risk of injury from the invasive surgery or from complications therefrom.

Traditionally, the parts of these joints have been made from the same material. For example, in the field of medical implants, the most commonly utilized material has been cobalt-chromium alloys. These materials are advantageous for these uses because they are strong enough to withstand the day to day forces applied to them and they are light enough to be suitable as a replacement for the natural joint, among other suitable characteristics. However, the wear between the two parts has made the use of these devices, for long term applications, somewhat undesirable. One proposed solution has been to use different materials to construct the joint parts, wherein one material is tougher than the other material. This makes the replacement of a single part necessary instead of the replacement of both parts. However, an invasive surgery is still necessary to remove and replace the worn part and therefore, this solution still provides a substantial risk of injury to a patient.

Particularly characteristic and problematic of the heretofore discussed dissimilar joint component material designs is that one component wears at the expense of the other: corrosion and wear damage to the implant release metal ions from the implant components into adjacent body tissue. More often than not, these ions are incompatible with the body, and can thus lead to physical reactions such as, for example, inflammations, bone degeneration, healing disturbances and similar problems. Such implant degradations further contribute to, among other things, an increase in friction and sticking between two movable components of the implant, with corrosion and wear damage contributing to a decrease in the static, and especially the dynamic strength/stability of the implant.

In the context of a hip joint prosthesis, the aforementioned phenomenon has been well documented. Hip joint prostheses typically have a ball joint design that includes a cup-shaped bearing portion, called the acetabular cup, and a mating portion, which is typically a ball-shaped element, called the head. The head is articulated in the cavity of the cup to permit motion. In a full replacement hip joint prosthesis, the head is provided by removing the existing femur ball, and implanting a prosthetic head with a rod-like member referred to as the neck and stem which is anchored to the femur. In another design, known as a surface replacement prosthesis, the head is provided by resurfacing the existing femur ball with a covering, typically metal.

The cavity of the acetabular cup is typically defined by a layer of ultra-high molecular weight polyethylene polymer (UHMWPE). The useful lifetime of the prosthesis is affected by wear of this polyethylene cup (i.e., the UHMWPE). One mechanism of wear is abrasion caused by the motion of the head. This abrasion can liberate fine particles which initiates biological processes having a negative physiologic effect, and, ultimately leading to failure of the prosthesis.

It should be readily appreciated although discussions to this point have been focused upon movable joints (e.g., a hip joint prostheses), that more generally, work pieces which are susceptible to an unacceptable degradation (e.g., corrosion, abrasion, wear, etc.) due to environmental conditions (e.g., cooperative engagement of components thereof), are likewise candidates for formulation/manufacture/treatment such that operative longevity is enhanced, and functionality generally improved.

It is an object of the invention, to provide an improved movable joint having reduced wear between the joint surfaces, such as both the ball and socket portions of a ball joint, and a mehtod of producing a coated ball joint.

This object is achieved by the method and the devices of the independant claims. Advantageous embodiments of the invention are characterized in the sub-claims. More particularly, to an improved movable joint having an extremely hard and dense, low friction, non-magnetic outer surface portion, a coating for a work piece susceptible to corrosion, wear damage, etc., and a methodology for coating such a work piece.

The present invention offers a solution to the above problems by providing a portion of the joint constructed having a chromium interface surface that reduces wear between the joint surfaces, such as both the ball and socket portions of a ball joint, by virtue of its intrinsic hardness and lubricity. The present invention generally provides a first portion and a second portion with either the first portion or the second portion having a chromium outer surface. For example, one embodiment of the present invention generally provides a ball joint, having a ball portion comprising at least a deposition of chromium forming an outer surface of the ball portion. Alternatively, the socket portion may have a deposition of chromium forming an interface surface thereon. The ball portion is adapted to be rotatably captured within a defined area of the socket portion, thereby capturing the ball portion in the socket portion. In each embodiment, the chromium deposition forms an interface surface between the first and second portions.

In a particular embodiment, the chromium material utilized for deposition on either the first or second portion of a movable joint is comprised of hexavalent chromium. The chromium material may be in the form of an electro-chemically bound, thin deposit of chromium on the outer surface of the portion. In such an embodiment, the interior structure of the portion may be comprised of a cobalt-chromium based alloy. Furthermore, the chromium may be bonded to the outer surface of the portion by electro-deposition.

In a ball-type joint, the socket portion generally has an area constructed and arranged to receive the ball portion in a movable relationship within the confines of the defined area. In one embodiment, the socket portion of the joint is formed from ultra high molecular weight polyethylene. This material provides a suitable and complimentary surface to that of a chromium deposited ball portion, thereby providing increased wear resistance to the device.

The features provided above may be combined to provide an embodiment comprising a joint having a first portion, formed of a cobalt-chromium based alloy, with an outer surface coated with a hexavalent chromium deposition applied over its outer surface, and a second portion formed from an ultra high molecular weight polyethylene material.

One application that joints, constructed according to the present invention, are particularly suited for is use in replacement of natural human or animal joints, such as knee, ankle, elbow, shoulder, spine, etc. However, the devices may be useful in any medical or non-medical application that, among other criteria, requires a joint with good wear resistance. Joints fabricated according to the present invention are also suited for these applications because they provide a reduction in fretting. Fretting is the production of wear debris through the interaction between two or more parts. The reduction of fretting reduces the chance of osteolysis, which occurs when wear debris enters the bloodstream.

One preferred method of producing a coated ball joint, comprises the steps of: providing a socket portion having an area adapted to receive a ball portion of the ball joint and the forming of either the ball or the socket portion having at least an outer interface surface comprised of chromium, wherein the ball portion is adapted to be received and captured, such that the ball portion is capable of rotatable movement, within an area of the socket portion. The method may also include the step of capturing the ball portion within the area of the socket portion. In a ball-type joint, wherein the ball is the first portion and the socket is the second portion, the socket has an area constructed and arranged to receive the ball in movable relation within the confines of the defined area and the ball portion adapted to be rotatably captured within the defined area of the socket portion.

Furthermore, in a process of electroplating a metal work piece with thin dense chromium, the steps of submerging the metal work piece in a 35% sulfuric acid solution having about 4 ounces per gallon HF as ammonium biflouride salts, and subsequently submerging the metal work piece in a thin dense chromium plating bath having an initial direct current of about 3 volts, the amperages being at about 1.5-2.5 amps per square inch of cathode area, is provided.

The present invention may be utilized with any movable joint, but is particularly applicable to ball-type joints. A movable ball joint is typically comprised of two main parts; a ball portion and a socket portion. The socket is constructed to encapsulate more than half of the ball portion, thereby securing the ball portion in a movable relationship with respect to the socket.

The aforementioned benefits and other benefits including specific features of the invention will become clear from the following description by reference to the accompanying drawings.
FIG. 1 is a cut-away side view of a ball-type embodiment of the present invention wherein the socket has been attached to the bone surface of a patient;
FIG. 2 is a magnified cut-away side view of a portion of the ball of the implant of the embodiment of FIG. 1 showing the interface of chromium applied to the surface of the ball portion;
FIG. 3 is a cut-away side view of an embodiment of the present invention showing an interface of chromium applied to the surface of the socket portion;
FIG. 4 is a cut-away side view of the embodiment of FIG. 3; and
FIG. 5 is a cut-away side view of an embodiment of the present invention in assembled condition showing the interface of chromium applied to the surface of the ball portion;
FIG. 6 graphically illustrates Taber Abrasion Wear Resistance Test data for a variety of substrates/coated substrates;
FIGS. 7-15 graphically illustrate D-C magnetic characteristics of a variety of substrates/coated substrates; and,
FIGS. 16-18 illustrate a two-buss bar fixture for operatively retaining a work piece during plating.

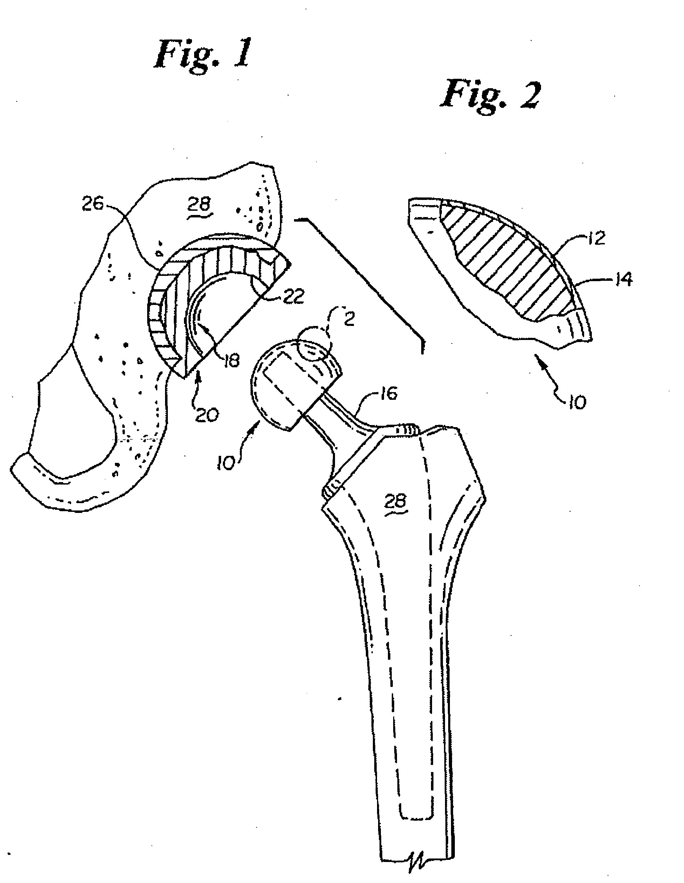

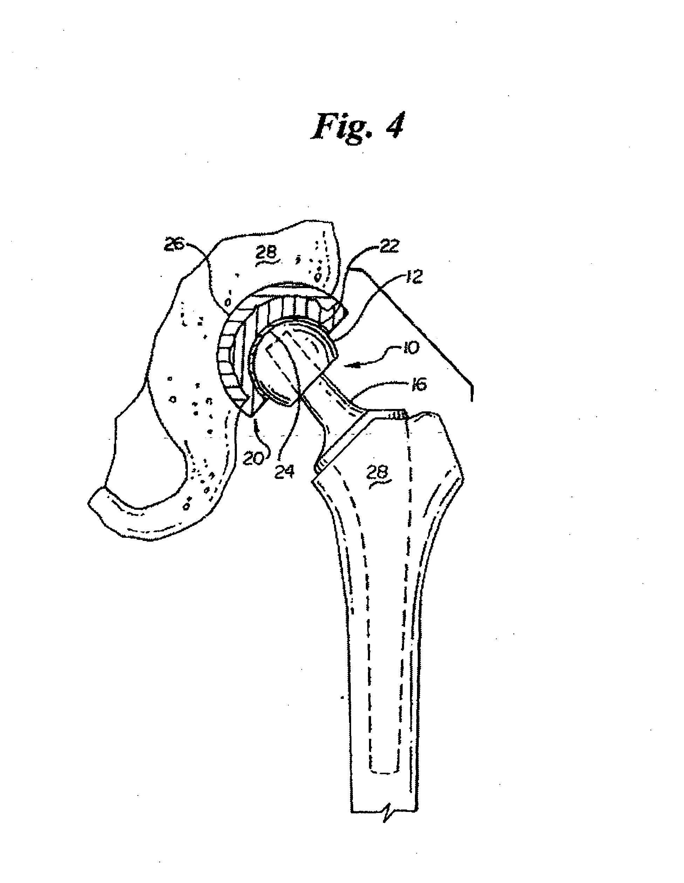

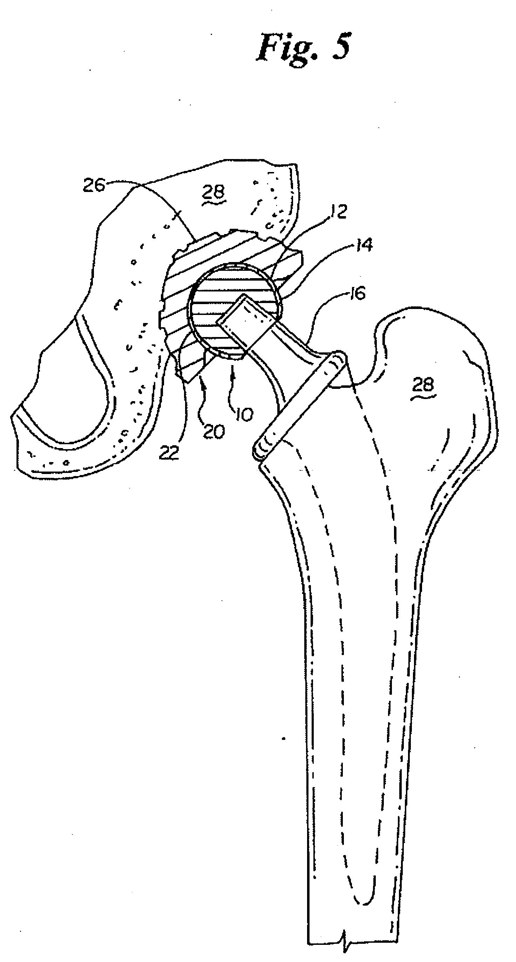

Referring now to the drawings wherein like reference numerals denote like elements throughout the several views, FIG. 1 illustrates a cut-away side view of an embodiment of the present invention. A ball-type embodiment of the present invention comprises a ball joint having a first, ball shaped, portion 10 having an outer surface 12 and a second, socket shaped, portion 20 having an outer surface 22. The ball portion 10 is sized and shaped to engage the cup 18 formed in the socket portion 20. As shown in FIGS. 4 and 5, the cup 18 is an area constructed and arranged to hold the ball portion 10 within the confines of the cup 18 and to allow the ball portion 10 to rotate within the confines of the cup 18. The ball portion 10 is typically attached to a stem 16 that is enabled to move relative to the socket portion 20 because of the rotatable engagement of the ball portion 10 with the socket portion 20.

The socket portion 20 and stem 16 of the ball portion 10 may be attached to an attachment surface 28 by any means known in the art. Some suitable examples of attachment means include: mechanical attachment assemblies, such as screws and nuts and bolts, and adhesive mechanisms, such as cement and glue, for example.

Furthermore, the shape of the surface 26 of the socket portion 20 utilized for attachment to the attachment surface 28 may be of any suitable shape known in the art. For example, FIGS. 1, 3, and 4 illustrate a socket surface 26 having a substantially uniform circular surface, whereas FIG. 5 illustrates a socket portion 20 having a non-uniform surface 26.

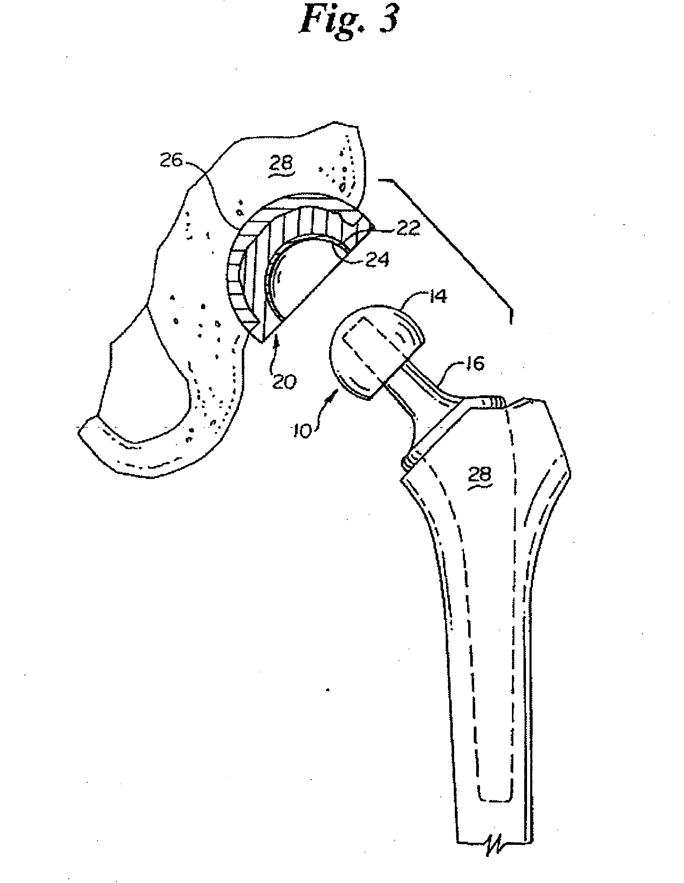

The surface coated with chromium material may be either the outer surface 12 of the first portion 10 or the outer surface 22 of the second portion 20. In the embodiment shown in FIGS. 1, 2, and 5 a thin deposition of chromium is placed over the outer surface 12 of the first portion 10. In the embodiment shown in FIGS. 3 and 4, a thin deposition of chromium is placed over the outer surface 22, generally formed within the cup 18, of the second portion 20.

By applying the chromium to one of the outer surfaces 12 or 22, the chromium provides an interface between the materials used to form the first and second portions. The interface may be utilized with any materials that form the first and second portions known in the art. For example, cobalt-chromium alloys or stainless steel are two examples of materials that may be coated with chromium within the purview of this invention.

Additionally, in a preferred embodiment of the present invention, when one of the first or second portions is coated with chromium, the other first or second portion may preferably be constructed from an ultra high molecular weight polyethylene material. For example, in one embodiment, a ball portion may be comprised of a cobalt-chromium alloy coated with a deposition of chromium and a socket portion may be constructed from ultra high molecular weight polyethylene. In another embodiment, both the first and second portions may be formed of a cobalt-chromium based alloy with one of the surfaces of the two portions having a chromium deposition thereon. As indicated above, the present invention may be provided on joints having both portions made of a single material, for example for a joint having both the first and second portions of the joint formed from metal.

It is also preferred that the chromium utilized for the deposition process be hexavalent chromium and that the deposition be electro-chemically bound. The chromium may be deposited through any process known in the art, such as electro-deposition. The deposition may occur by flash coating the surface, thereby depositing the chromium thereon. One suitable thickness for the chromium deposition is approximately 2/10,000 of an inch, however, the deposition may be as small as 50/millionths of an inch. The process of applying the coating may also include pre and post plating mechanical polishing.

The coating of the subject invention is a precisely controllable, extremely hard and dense low-friction, non-magnetic, 100% chromium coating. Work piece coating utilizing the methodology of the subject invention results in a smooth, fine grained deposit that is uniform in thickness and appearance, the surface free of blisters, pits, nodules and porosity, with minimal edge buildup. A detailed discussion of the coating, and work pieces so coated, more particularly, the advantageous features, physical properties, and biological properties thereof, immediately follows, with a presentation of the attendant coating methodology thereafter.

The coating of the subject invention is uniformly deposited on metal work pieces or substrates. Generally, the coating is applied directly to the base metal without an intermediate coating. It is preferably applied following completion of all base metal processing, including, but not limited to, machining, brazing, welding, heat treating, and stress relieving. The coating greatly improves the appearance, performance and service life of, among other things, medical devices. The coating increases resistance to wear, maintains sharpness of edges, prevents galling, and seizing, minimizes corrosion, and provides a smooth surface that is easy to clean.

Advantageously, the subject coating can be applied with great precision and consistency. The practical coating range (i.e., applied thickness) for the subject coating is 0.000025 inches - 0.0006 inches (0.64 microns - 15.38 microns). Depending upon the thickness specified, and the part's quality requirements, the following thickness tolerance can be maintained: +/- 0.000010 inch to +/- 0.000050 inch (+/- 0.25 microns to +/- 1.28 microns). The following preferred thicknesses are noted for the following typical applications: (1) cutting surfaces; 0.00005 inches - 0.0001 inches (0.25 microns - 2.56 microns); (2) light wear; 0.0001 inch - 0.0003 inch (2.56 microns - 7.69 microns); and (3) severe wear; 0.0003 inch - 0.0005 inch (7.69 micron - 12.82 micron).

As to adhesion, articles coated utilizing the subject methodology can be repeatedly bent and twisted without chipping, flaking, or otherwise separating from the substrate. Articles coated with the subject coating show no evidence of discoloration, cracking, flaking, rust or other change following repeated autoclave exposures. As will be later discussed in detail, the subject coating increases the surface layer hardness of uncoated steel, for example, the hardness of the coating, as applied to laboratory samples, is Rc72. The roughness average (Ra) of the subject coating, when measured in accordance with ASME B 46.1-1995 will not significantly vary from the Ra of the part prior to coating. Both internal and external surfaces of virtually all shapes and configurations can be uniformly coated. All grades of stainless steel may be processed utilizing the subject methodology, furthermore, the subject coating and methodology may be practiced upon most ferrous metals, and some non-ferrous metals, such as copper and aluminum. Finally, as to biocompatibility, the subject coating meets or exceeds USP Class VI Certification.

Physical property testing of the subject coating has been conducted, more particularly, testing specific to: wear resistance, crevice corrosion, microhardness, composition of coating, embrittlement relief, resistivity, magnetic characteristics, adhesion to base metal, and autoclavability. Generally, two types of stainless steel substrate were used in testing the subject coating: AMS 5511 (low C, 18% Cr, 8% Ni steel: sheet; AMS 4 SAE 30304), hereinafter Type I, a/k/a, AISI type 304 stainless steel; and, AMS 5504 (0.15% C, 12.5% Cr steel: sheet; AMS 4 SAE 51410), hereinafter Type II, a/k/a, AISI type 410 stainless steel.

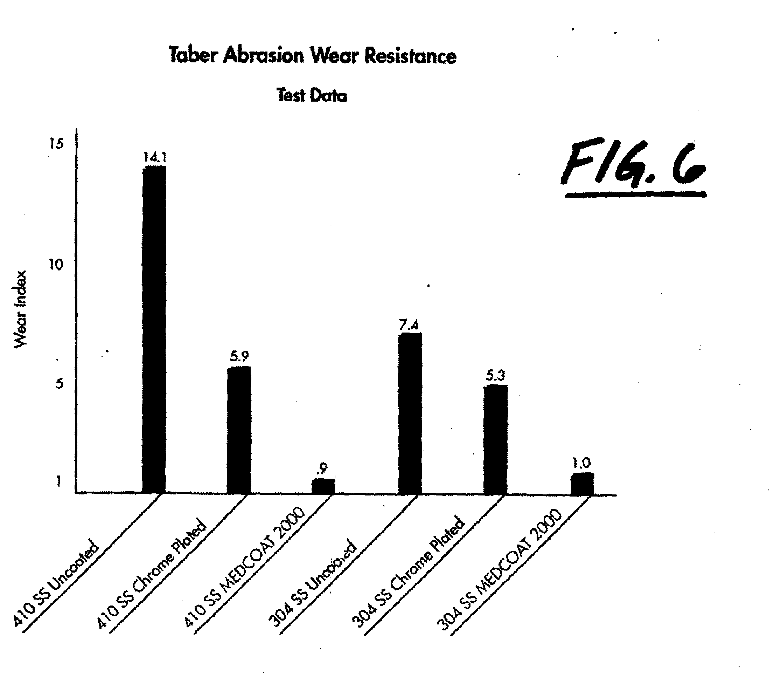

As to abrasion testing, one of each material type of substrate was coated with the coating of the subject invention, one was coated with conventional hard chrome, the other was uncoated. Abrasion testing was performed in compliance with Specification FED-STD-141, Method 6192.1; CS-10 Calibrase Wheels were used; 1,000 grams of pressure was used against the test panels; and, all panels were cleaned with acetone prior to weighing. Taber abrasion wear resistance (i.e., a wear index) is illustrated in FIG. 6 for the test coupons, more particularly, the wear index for the 410 stainless coupons (uncoated, conventional hard chrome plated, and bearing the coating of the subject invention) and 304 stainless (uncoated, conventional hard chrome plated, and bearing the coating of the subject invention) are displayed left to right. For each substrate (i.e., 410/304 stainless), the coating of the subject invention yielded an improved wear resistance over it, and the conventionally chrome plated version of same, more particularly, the wear index of the uncoated substrate was about 15.7 and 7.4 times greater than the same substrates (i.e., 410/304 stainless) bearing the coating of the subject invention, respectively, the wear index of the conventionally coated substrates being about 6.55 and 5.3 times greater than the same substrates bearing the coating of the subject invention, respectively.

A microhardness evaluation was had of two test specimens in accordance with ASTME3, E384, E140 and B487. Two specimens of one type of material were used in the testing, namely, the Type II stainless. One of the two specimens was coated with conventional hard chrome, the other was coated with the coating of the subject invention. Results of the microhardness survey are herewith as TABLE I. Both Knoop and Vickers test results indicate that the microhardness of the coating of the subject application specimen is greater than that of the conventional hard chrome plated specimen (i.e., 73 average HRC versus 72 average HRC, and 70 average HRC versus 67 average HRC for the Knoop and Vickers tests respectively).

Four V-notched tensile specimens coated with a 0.015 mm layer of the coating of the subject invention were subject to the 200-Hour Hand Test for Hydrogen Embrittlement Susceptibility, in accordance MIL-STD-1312/5A. The rack load was set at 2,799.83 kg. Temperature at loading time was 23.66° C. The set-up was loaded at 75% ultimate strength. The specimens hung for 263.2 hours without breaking, thereby meeting the 200 hour minimum requirement.

As to electrical resistance, the Type I stainless steel was used in the testing, with one such test specimen coated with the coating of the subject invention, the other specimen not coated. Testing was performed using a recently calibrated Valhalla Scientific Digital Micro-Ohmmeter, Model 4300b. The testing of electrical resistivity was conducted in a straight forward manner, contact probes were placed in various areas of the coated and uncoated panels, the test temperature was 21.66° C. Resistance measurement data is presented in TABLE II.

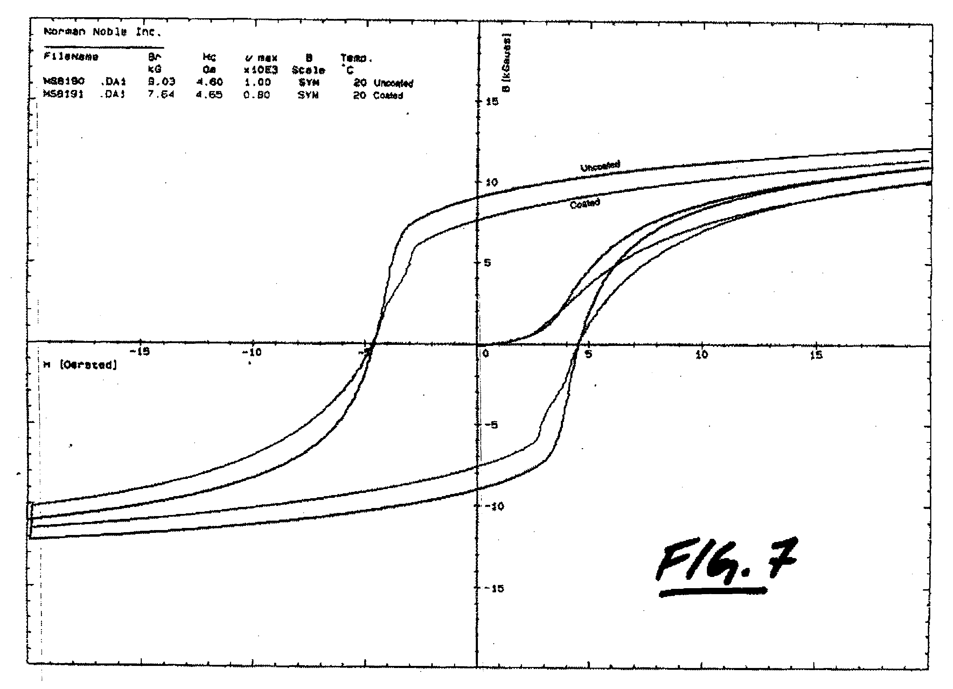

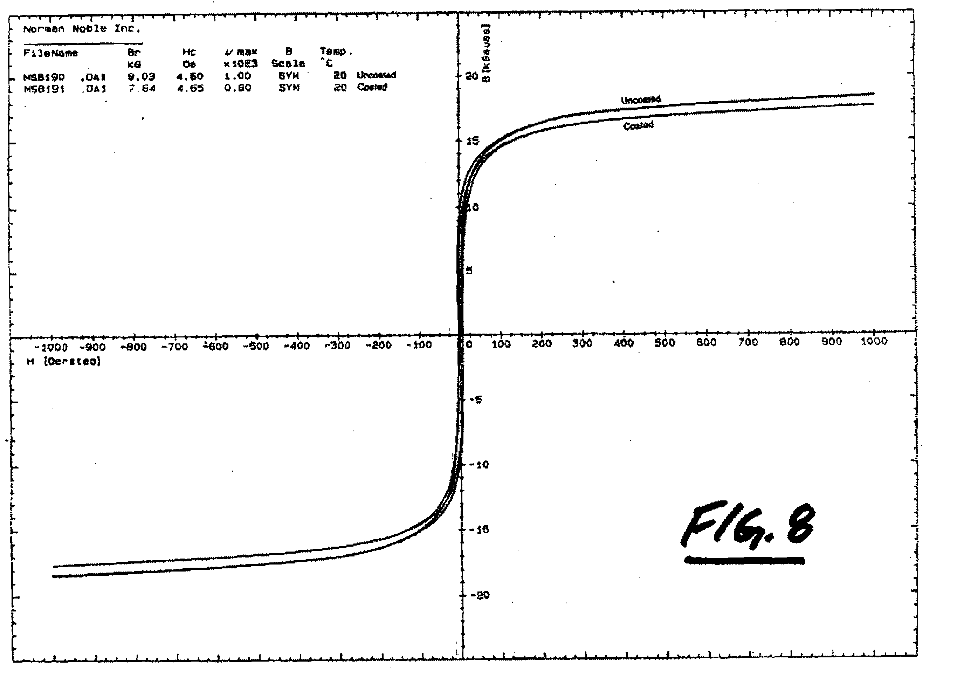

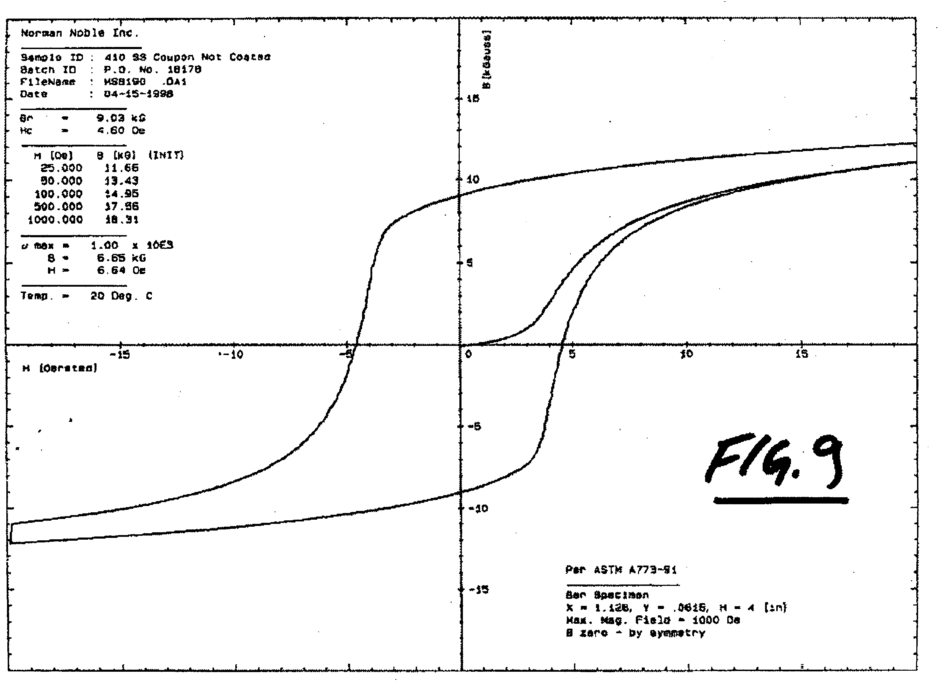

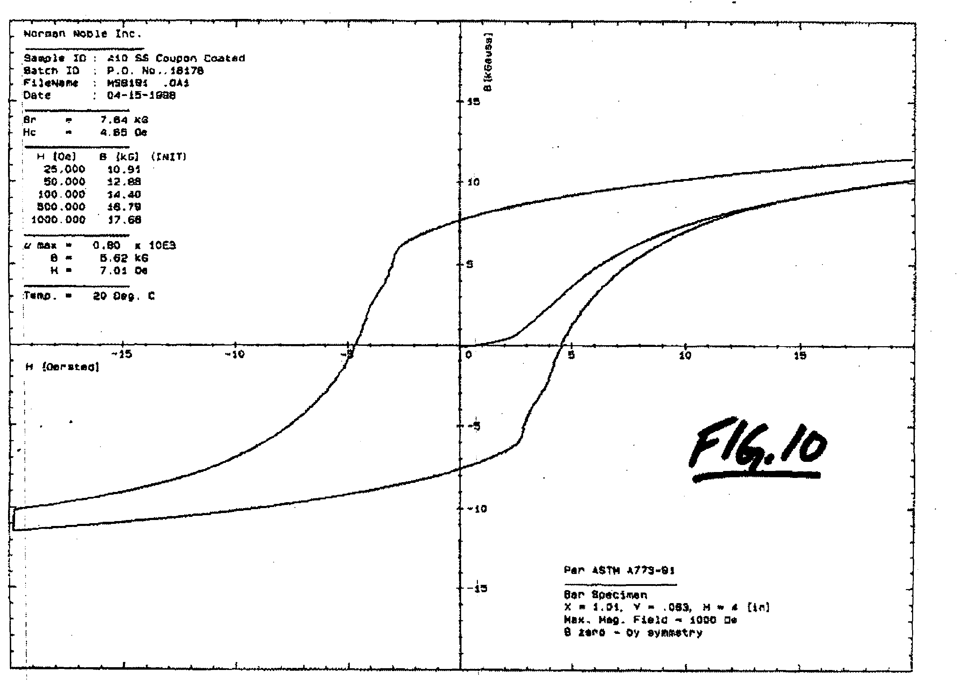

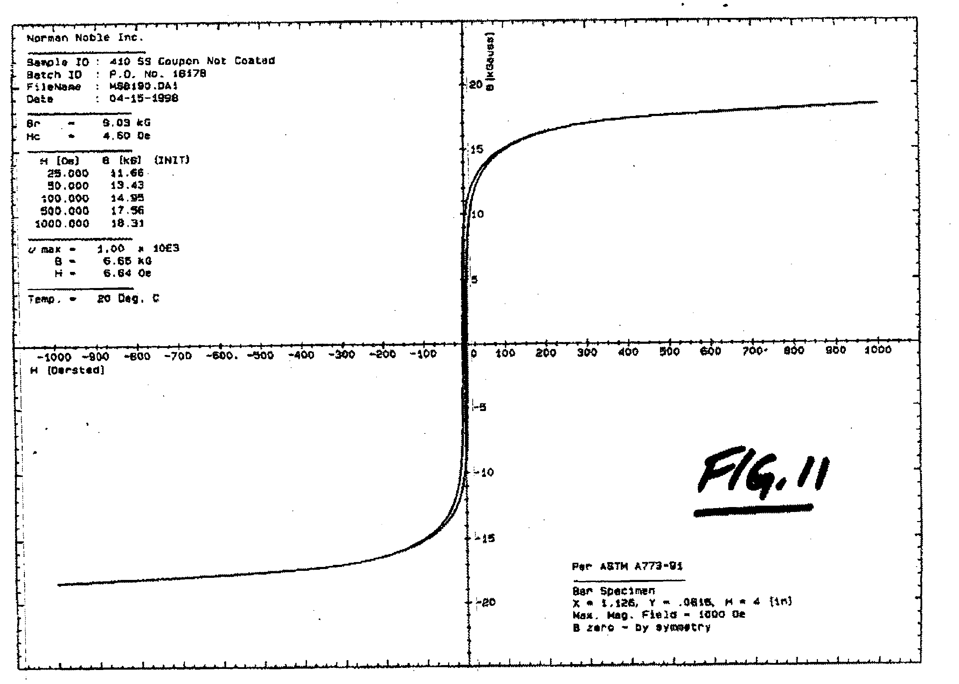

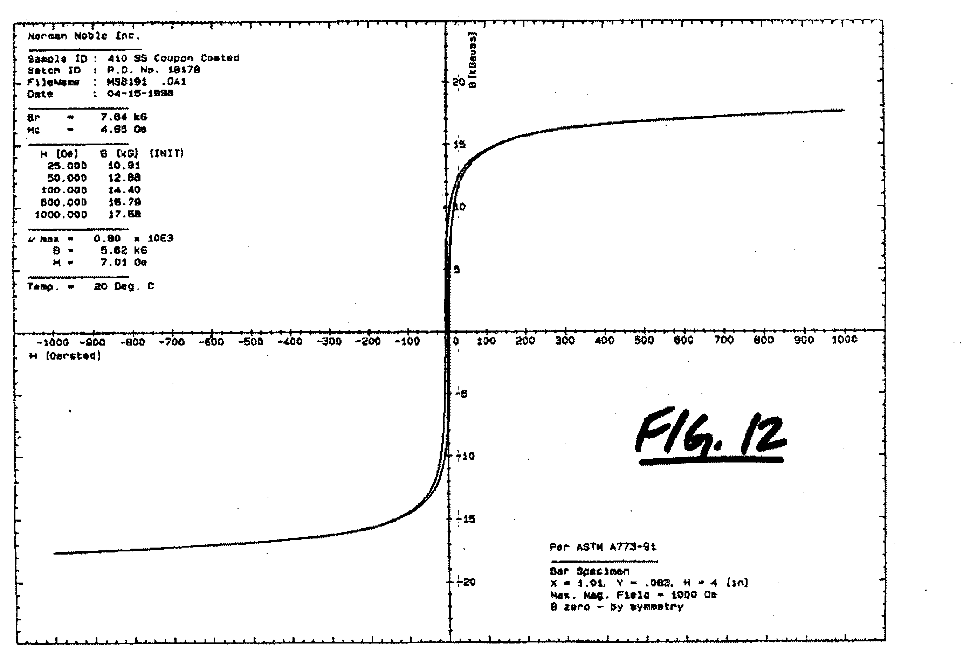

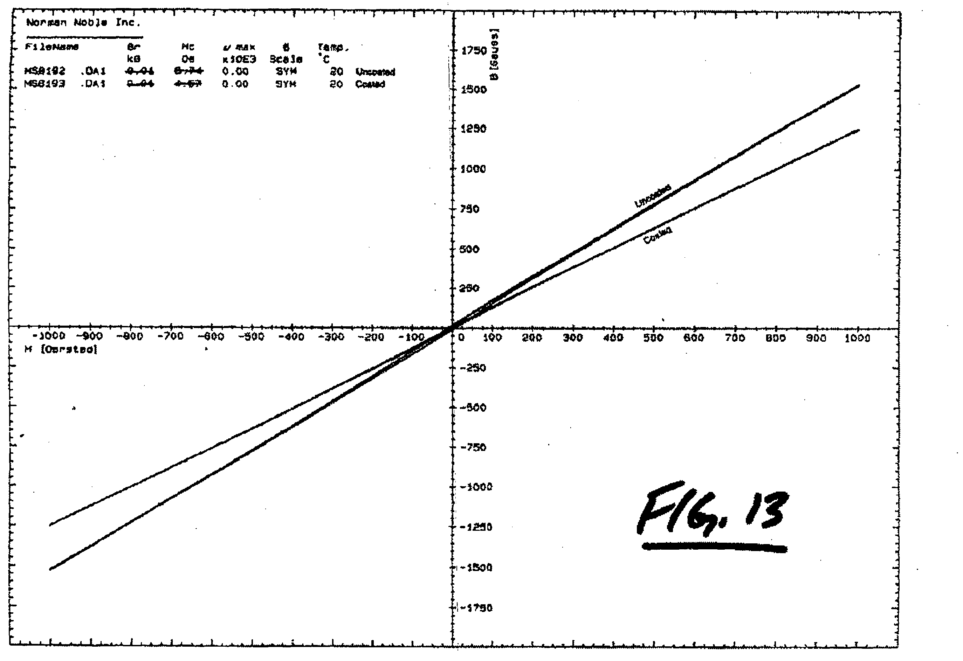

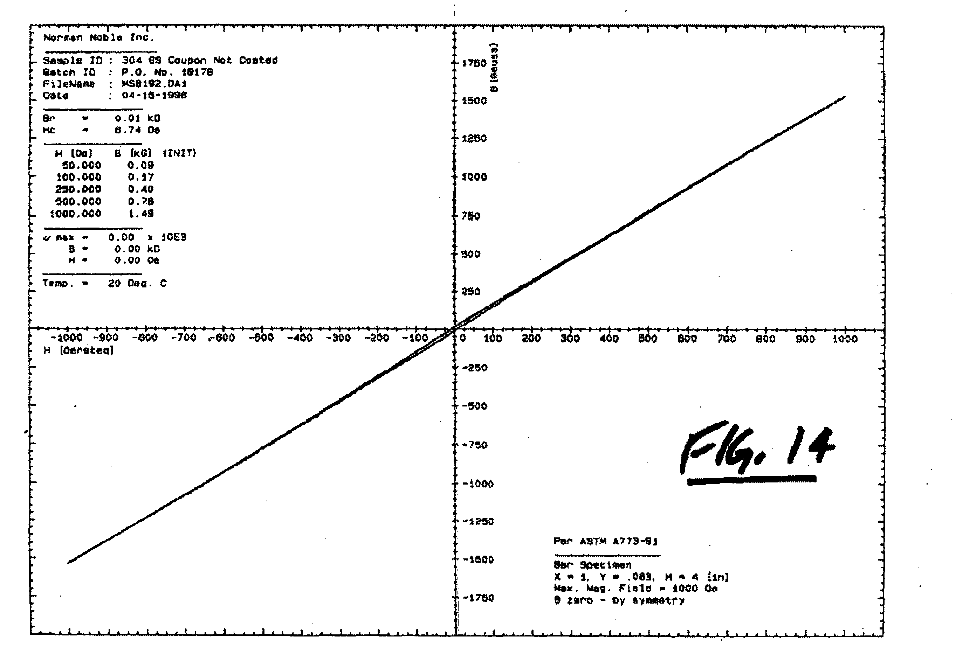

As to D-C magnetic characteristics (B, H curve), two types of stainless steel were used in the testing, namely those as previously specified. One specimen of each material type was coated with the coating of the subject invention, the other specimen of each type was not coated. Testing equipment and procedures complied with ASTM A773-96, "Standard Test Method for D-C Magnetic Properties of Materials Using Ring and Parameter Procedures with D-C Electronic Hysteresigraphs." Basically, a magnetic field is applied to the specimen, and the material is tested to determine how much magnetism is retained in the material once the magnetic field is withdrawn. Depending upon its magnetic characteristics, a material can be classified as: diamagnetic, namely having extremely weak magnetic properties; paramagnetic, namely having generally weak magnetic properties; and, ferromagnetic, namely having strong magnetic properties, such materials including iron, cobalt and nickel. Metal chromium is classified as a paramagnetic material. Generally, this class of materials is weakly magnetic in nature. Typically, materials that are paramagnetic and diamagnetic are described as non-magnetic, in contrast to strongly magnetic ferromagnetic materials. The graphical test data of FIGS. 7-15 support a conclusion that the specimens coated with the coating of the subject invention were less permeable to a magnetic field compared to an uncoated specimen of the same material.

As to adhesion testing, one specimen of the Type I stainless was coated with a 0.015 mm thickness of the coating of the subject invention. Testing was performed in compliance with specification ASTM B571. This specification requires bending the specimen through an angle of 180° until failure. Upon failure, the broken ends of the specimen were examined at 10X magnification, with no separation or pealing of the coating noted.

As to autoclavability, two samples, placed in a heat-resistant dish, were subject to over 40 autoclave cycles. Steam exposure was conducted in production type, gravity displacement steam sterilization vessels at 120°F (50° C). After every fifth cycle, visual microscopic inspections were made for discoloration and degradation of the samples. Tests were discontinued because repeated autoclave exposures had no effect on the outer surface of the samples.

Finally, as to biological compatibility, a summary of test results is provided in TABLE III. The implants coated with the coating of the subject invention have passed ISO/Tripartite testing, and are USP Class IV Certified.

As to the available coating methodologies, electroplating is a well known technique for coating a metal or plastic surface with a metal. An article so coated has a surface which is brighter and more corrosion resistant than the substrate to which the coating is applied. Electroplates are generally applied by immersing a work piece to be coated in a tank containing select chemicals dissolved in water (i.e., a plating bath). The work piece to be plated is attached to a negative electrical lead, and thus becomes a cathode. The other electrical lead, the positive electrical lead, is in the solution (i.e., the anode). When current is supplied to the plating solution, the negatively charged immersed work piece attracts the positively charged metal from the solution. This continues as long as current is on, with the coating or deposit becoming thicker and thicker as a function, among other things, time.

In chromium plating baths, in addition to a chromium source, sulfate and fluoride ions may be introduced so as to act as catalysts. Temperature, current density, and bath composition affect the film characteristics and current efficiency. These parameters are therefore carefully controlled in order to obtain specific deposit properties, and plating rates. As to bath compositions, chromic acid and sulfate are the necessary ingredients. Generally, chromic-to-sulfate ratios range from 75:1 to 250:1. The specific composition is primarily dependent upon whether the bath is co-catalyzed, e.g., with fluoride, fluorosilicates or fluoroboron. Hexavalent chrome is the source of chromium deposited from such baths, with chromic acid being the main component in the solution make up. During the general process, hexavalent chrome is first reduced to trivalent chrome, is next reduced to the unstable divalent state, and further and final reduced to the stable, zero valence state (i.e., elemental chromium).

Plating bath temperature is closely related to current density and its affect on brightness and coverage of deposit. Generally, the higher the current density, the higher the temperature requirement. An optimum temperature range generally exists for a given concentration of chromic acid. Below or above that range, dull deposits result. For hard chromium, the range is 120°F (49°C) to 150°F (65.5°C). Preheating of parts to optimum bath temperature may be needed before they are introduced into the plating tank, and in rare instances, cooling of parts may required, in order to ensure uniformity of deposit.

At a given solution composition temperature, current density affects cathode efficiency, brightness and hardness. Too-high current densities result in burning or roughness of deposition, whereas, at low current densities, lack of chromium coverage can be expected.

Self-regulating high-speed chromium baths incorporate fluoride complexes such as silicofluoride, in addition to sulfates. Salts of low solubility are selected and used to release the desired anions on a controlled basis. Mixtures containing potassium or sodium silicofluoride and dichromate, for example, regulate the release of fluoride via the common-ion affect. Mixtures of strontium sulfate and chromate regulate the release of sulfate in solution. Consequently, at higher temperatures, the cathode current efficiency increases as a result of the increased solubility of catalysts in this type of bath.

As to the preferred electroplating bath composition of the subject invention, the following commercially available products are preferred; HEEF-25 hard chrome plating solution, 30-35 once per gallon chrome, 0.3-0.35 once per gallon sulfuric acid, balance water heated to 135-140°F; Oakite 90 alkaline cleaner, 8-12 once per gallon, heated to 90-105° F; 35% sulfuric acid, 4 once per gallon HF (as ammonium bifluoride salts) at ambient temperature (i.e., 65-80° F); and, McGean Rohco solution at 4 once per gallon (rinse-aid). The coating methodology of the subject invention utilizes conventional electroplating equipment and commercially available chemicals. Select process elements preferably include: a poly-lined steel plating tank with air agitation; quartz heaters (5,000-watts); a temperature control unit, including thermostat and thermocouple; a rapid rectifier having a DC 480 3-phase input, 0-9 volt DC output, less than 5% ripple; a steel tank for cleaning process equipped with 2,000-watt electric heaters and temperature control; triple stage cold water rinse tanks; poly acid clean tanks; and, a plating fixture.

The coating methodology or procedure of the subject invention is presently presented in the context of processing cobalt chrome implants. Generally, the implants are received at the plating facility in sterile packaging with strict lot control identification serial numbers. Traceability must be maintained during all phases of coating. Prior to opening the packaged implants, a work router is prepared. A 9 inch by 16 inch by 2 inch deep poly container, lined with clean bubble wrap and provided with a snap lock lid, is preferably utilized to protect the implant as it is moved from inspection to production areas. The router will stay with the implant during processing.

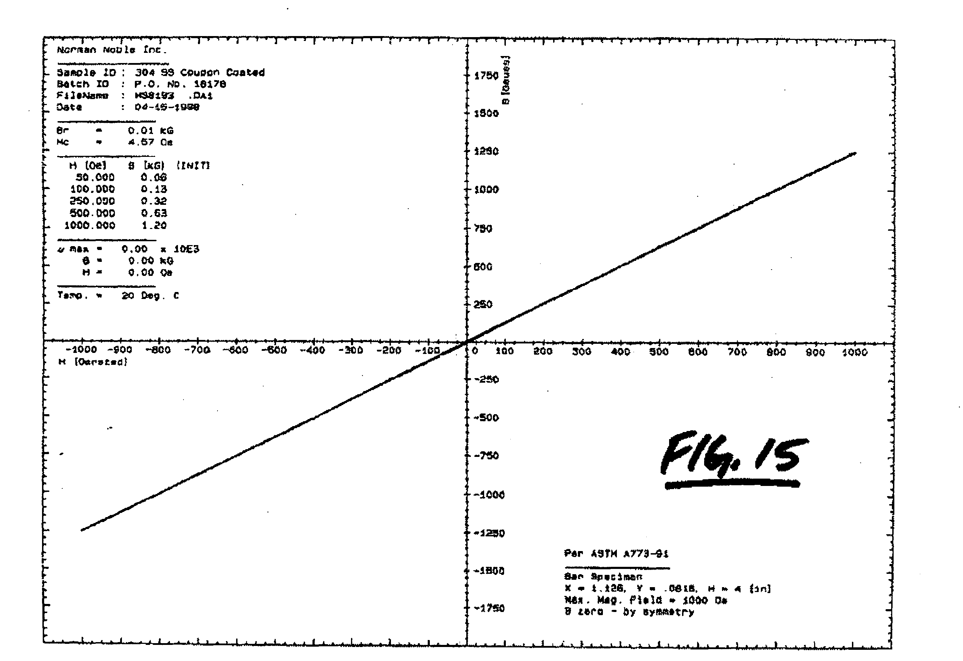

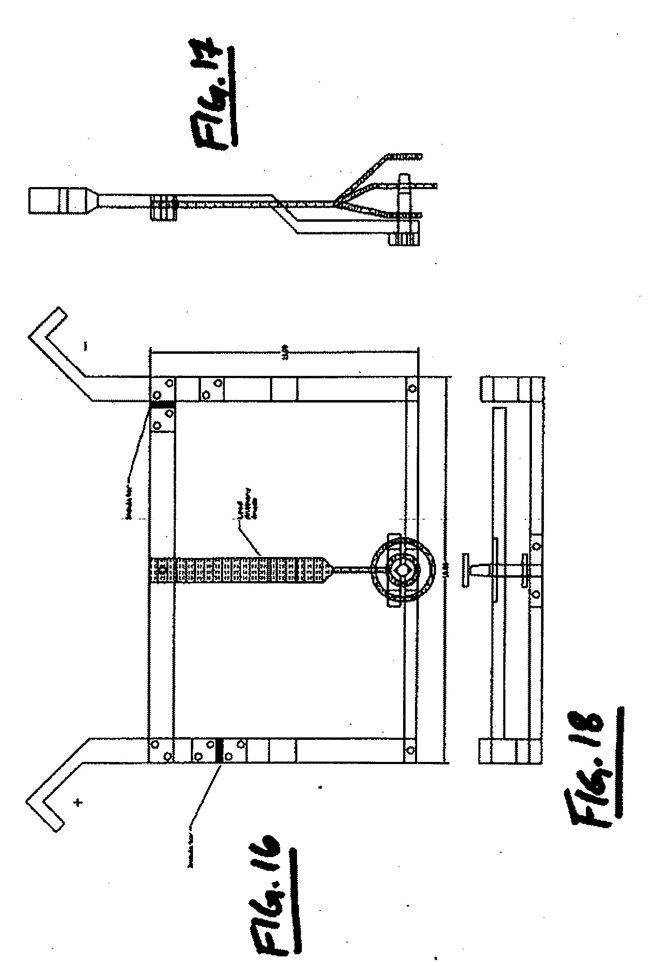

The container, router and implant is taken to the plating production area, where an operator removes the implant from the container while wearing white lint free gloves. The implant is placed upon the cathode of a two-bus bar fixture, see for example FIGS. 16-18. The implant is next wiped with a lint free rag soaked in rinse aid solution at room temperature (i.e., between about 65-75°F). The anode is subsequently positioned such that no more than one inch of spacing is present between the anode and cathode. The plating fixture is next rinsed with cold clean running water, and is thereafter submerged to sufficient depth in alkaline cleaning solution to cover the implant with about two inches of the cleaning solution. The rack will remain submerged in the solution for approximately two minutes, while gently agitating the fixture by hand. The fixture is subsequently removed in cold, clean running water.

The fixture is subsequently submerged in an acid cleaning bath so as to allow about two inches or more of solution to cover the implant. The fixture is anodically activated for approximately 30 seconds at about 3 volts direct current, allowing approximately 2-3 amps per square inch of cathode area. The plating fixture is next rinsed with cold clean running water, and the fixture is subsequently submerged into a thin dense chrome plating bath, with the implant being the cathode, the DC current being on, and set to about 3-3.5, and most preferably, 3.2 volts. The DC current is to be selectively adjusted upward, at the rate of approximately 0.1 volt every 10 seconds, until a voltage of approximately 4-5 volts, and preferably 4.5 volts, is achieved. The amperage will be noted and calculated to be within a range of about 1-3 amps, and a most preferred range of about 1.5 to 2.5 amps, per square inch of cathode area. A plating rate (i.e., rate of deposition) of 0.0001 inches every 6 minutes results, requiring a dwell time of approximately 48 minutes to deposit a chromium coating of 0.0008 inches minimum thickness. At the end of the plating run, the fixture will be removed from the plating bath, and rinsed in a triple stage return rinse tank.

The fixture will next be forced-air dried, and the implant removed from the cathode of the fixture. The implant will be hot and cold water rinsed no less than three separate times to remove any residual chrome solution. The implant will thereafter be examined by the operator for any stains or discoloration on any of the internal or external surfaces of the implant. Soaking in clean, hot water and wiping with a lint free cloth will remove any stain or discoloration. All cleaning operations must be performed within five minutes of removal from the plating solution.

The implant is next placed back into the original handling container, with all documentation completed by the operator. The closed container will then be transported to the inspection area for final examination for thickness, uniformity of coating, and cleanliness. Thereafter, the implant will finally be lapped, polished and inspected for uniformity of coating and acceptable surface finish, and repackaged in the original sterile package, with all documentation attached thereto for traceability.

## Claims

1. A process of electroplating a metal work piece with thin dense chromium, comprising:
a. submerging the metal work piece in a 35% sulfuric acid solution having about 4 ounces per gallon HF as ammonium biflouride salts; and,
b. subsequently submerging the metal work piece in a thin dense chromium plating bath having an initial direct current of about 3 volts, the amperages being at about 1.5-2.5 amps per square inch of cathode area.

2. The process of claim 1 wherein the metal work piece is anodically activated while submerged in said sulfuric acid solution.

3. The process of claim 2 wherein the anodic activation comprises delivery of about 3 volts direct current for about 30 seconds.

4. The process of claim 3 wherein about 2-3 amps per square inch of cathode area are provided during anodic activation.

5. The process of any of the claims 1 to 4 wherein said initial direct current voltage is selectively increased over time, in particular incrementally increased over time.

6. The process of claim 5 wherein said initial direct current voltage is increased to a maximum of about 4.5 volts.

7. The process of claim 6 wherein said initial direct current voltage is increased at a rate of 0.1 volt every ten seconds until a voltage of about 4.5 amps is achieved.

8. A coated implant produced by thin dense chromium plating a work piece, said coated implant having a 100% chromium coating, and a Tabor Abrasion Wear Resistance Wear Index in the range of about 0.9-1.0.

9. The coated implant of claim 8 wherein said work piece comprises a metal article, in particular a stainless steel article, in particular a 304 stainless steel or 401 stainless steel article.

10. The coated implant of claim 9 wherein said work piece comprises a cobalt-chromium article, a copper article or an aluminum article.

11. A coated implant produced by thin dense chrome plating a work piece, said coated implant having a 100% chromium coating, and a Knoop Test microhardness value in excess of 72 HRC.

12. A coated implant produced by thin dense chromium plating a work piece, said coated implant having a 100% chromium coating and a Vickers Test microhardness value in excess of 67 HRC.

13. In a process of electroplating a metal work piece with thin dense chromium, the steps comprising:
a. submerging the metal work piece into a thin dense chromium plating solution comprising chromium in the range from about 30 to 35 ounces per gallon of said solution, and sulfuric acid in the range from about 0.3 to 0.35 ounces per gallon of said solution, said solution being at a temperature of about 135-140° Fahrenheit, and carrying a direct current voltage of about 3.2 volts; and,
b. upwardly adjusting said direct current voltage at a rate of about 0.1 volt every ten seconds until a direct current voltage of 4.5 is achieved.
